Europäisches Patentamt

European Patent Office

Office européen des brevets

(11) Veröffentlichungsnummer: **0 143 280**
**B1**

(12) **EUROPÄISCHE PATENTSCHRIFT**

(45) Veröffentlichungstag der Patentschrift:
13.01.88

(51) Int. Cl.⁴: **B 01 J 8/04,** C 01 C 1/04,
C 07 C 29/15

(21) Anmeldenummer: **84111888.8**

(22) Anmeldetag: **04.10.84**

(54) Vorrichtung zur Durchführung exothermer, katalytischer Gasreaktionen für die Ammoniak- oder Methanol-Synthese.

(30) Priorität: **29.11.83 DE 3343114**

(43) Veröffentlichungstag der Anmeldung:
**05.06.85 Patentblatt 85/23**

(45) Bekanntmachung des Hinweises auf die Patenterteilung:
**13.01.88 Patentblatt 88/2**

(84) Benannte Vertragsstaaten:
**AT BE CH DE FR GB IT LI NL SE**

(56) Entgegenhaltungen:
**EP-A-0 121 355**
**DE-A-3 026 199**
**DE-A-3 146 778**
**FR-A-2 343 699**

(73) Patentinhaber: **Uhde GmbH, Friedrich- Uhde-Strasse 15, D-4600 Dortmund 1 (DE)**

(72) Erfinder: **Förster, Friedrich, Dipl.- Ing.,
Rauschenburgstrasse 1, D-4600 Dortmund 30 (DE)**
Erfinder: **Brieke, Hans- Günter, Dipl.- Ing.,
Godekinstrasse 55, D-4600 Dortmund 30 (DE)**
Erfinder: **Marsch, Hans- Dieter, Dipl.- Ing.,
Overhoffstrasse 193, D-4600 Dortmund 76 (DE)**

## Beschreibung

Die Erfindung betrifft eine Vorrichtung zur Durchführung exothermer, katalytischer Gasreaktionen für die Ammoniak- oder Methanol-Synthese, im wesentlichen bestehend aus einem Hochdruckmantel und einem Einsatz mit oberem Abschlußdeckel, wobei der Einsatzmantel mindestens zwei übereinander angeordnete Katalysatorbehälter in ringförmiger zylindrischer Anordnung mit gasdurchlässiger Innen- und Außenwand für radiale Gasströmung von außen nach innen und zwei Gas/Gas-Wärmetauscher in Rohrbündelbauart zentral innerhalb des ersten und des zweiten Katalysatorbehälters enthält.

Die katalytische Hochdrucksynthese zur Erzeugung von Ammoniak und Methanol ist ein exothermer Prozeß. Man ist seit jeher bestrebt, diesen Prozeß bei solchen Temperaturen ablaufen zu lassen, daß die thermischen Verhältnisse zu optimaler Reaktion, d.h. zu optimalem Umsatz führen. Die Einhaltung der günstigsten Reaktionstemperaturen geschieht durch Abkühlung der bei der Reaktion sich stark erwärmenden Reaktionsgase. Da das Frischgas mit einer Temperatur von über 300°C auf die 1. Katalysatorschicht gegeben werden muß, wird angestrebt, das Frischgas mit dem heißen Reaktionsgas in indirektem Wärmetausch aufzuheizen.

Die Abkühlung des Reaktionsgases kann nun entweder so erfolgen, daß die Reaktion fast isothermisch verläuft, wie es bei Katalysatorröhrenöfen oder Vollraumöfen mit Kühlschlangen in der Katalysatormasse der Fall ist, oder so, daß die Abkühlung stufenweise nach einzelnen Katalysatorpackungen erfolgt, in die die gesamte Katalysatormasse aufgeteilt ist.

Es sind zahlreiche Vorrichtungen bzw. Ofentypen bekannt zur Lösung der o.g. Forderungen. Es ist beispielsweise bekannt, die Katalysatormasse in mehreren Lagen untereinander anzuordnen und in den Zwischenräumen Röhren-Wärmetauscher für aufzuheizendes Frischgas einzubauen um die Reaktionstemperaturen zu senken.

Nach DE-A 30 26 199 ist ein Axial-Radial Reaktor mit zentral angeordneten Zwischenwärmetauschern bekannt. Dabei strömt das kalte Frischgas von unten nach oben rohrseitig nacheinander durch die einzelnen Zwischenwärmetauscher und das heiße Reaktionsgas mantelseitig um die Rohre. Jeder Zwischenwärmetauscher ist zentral im unteren Teil des ringförmigen Katalysatorkorbes angeordnet. Katalysatorkorb und Wärmetauscher sind so eingebaut, daß sich jeder Wärmetauscher nur ausbauen läßt, wenn zuvor der entsprechende Katalysatorkorb geleert und ausgebaut wird. Darüber hinaus ist ein elastisches Zwischenglied zwischen den einzelnen Wärmetauschern erforderlich, das erst gelöst werden muß bevor der nächste Zwischenwärmetauscher im Schadensfalls ausgebaut werden kann. Weiterhin ist es nicht möglich, bei Auswechslung des Katalysators diesen nach unten aus den Reaktor zu entfernen. Die Tatsache, daß zuerst der Katalysatorkorb entfernt werden muß bevor ein Wärmetauscher ausgebaut werden kann, zwingt dazu, den Abschlußdeckel des Hochdruckmantels über den vollen Querschnitt auszulegen. Ein eingezogener Deckel kann nicht verwendet werden.

Andere allgemein bekannte Ofentypen mit eingebauten Wärmetauschern in der bisherigen Bauweise in den Schichten haben allgemein den Nachteil, daß sie komplizierte und schwierig auszuwechselnde Einrichtungen benötigen und Abdichtungsschwierigkeiten bei den großen Temperatur- und Druckdifferenzen während des Betriebes einer Ammoniak- oder Methanol-Synthese auftreten.

Der vorliegenden Erfindung liegt die Aufgabe zugrunde, eine neue Reaktorkonstruktion zu finden, durch die Nachteile der bekannten Konstruktionen überwunden werden.

Die Aufgabe wird erfindungsgemäß gelöst durch eine Vorrichtung zur Durchführung exothermer, katalytischer Gasreaktionen für die Ammoniak- oder Methanol-Synthese, mit den Merkmalen entsprechend den konstruktiven Ausgestaltungen gemäß den Kennzeichen des Hauptanspruches.

Nach einer Ausgestaltung der Erfindung kann das zentrale Leitrohr auch ringförmig von der Zufuhrleitung für das Zumischgas umgeben sein, wobei die Anfangsrohrbodenplatte des zweiten Gas/Gas-Wärmetauschers die Zufuhrleitung für Zumischgas gasdicht und kraftschlüssig umschließt.

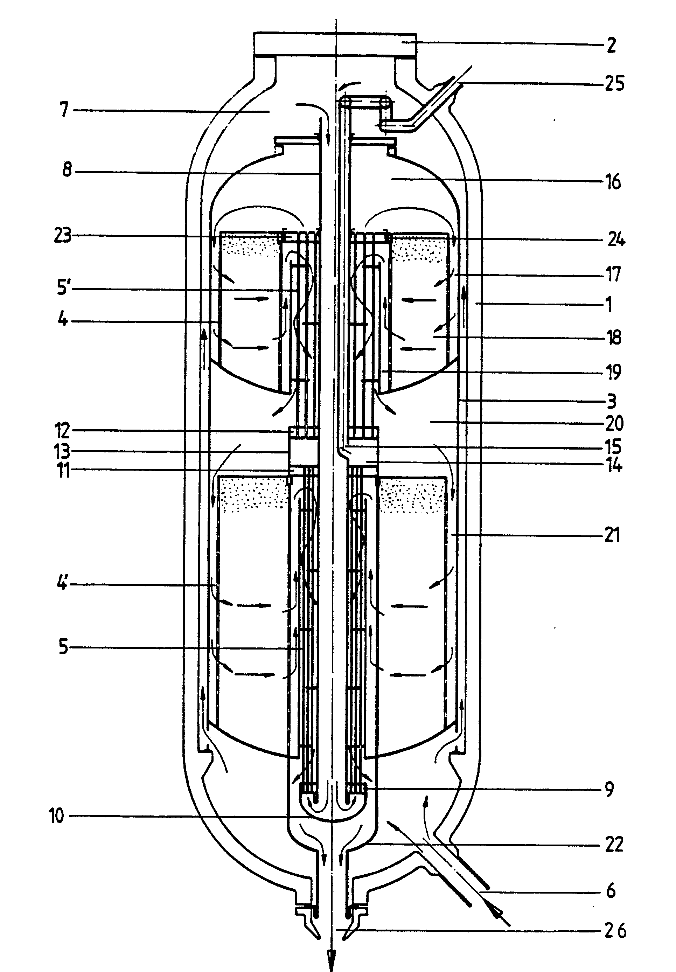

Die nachfolgend an Hand der Abbildung ausführlich beschriebene erfindungsgemäße Vorrichtung führt einmal zu optimalem Wärmetausch in den Ammoniakkonverter, da die Wärmetauscher mit optimalem Durchmesser/Längenverhältnis entsprechend den vorliegenden Gasmengen und erforderlichen Gasgeschwindigkeiten ausgelegt werden, zum anderen bringt sie eine konstruktive Vereinfachung des Einbaus von Wärmetauschern in Katalysatorschichten und vereinfachte betriebliche Handhabe des Ofeneinsatzes bzw. der Wärmetauscher.

Vor allem wird durch die erfindungsgemäße Reduzierung der Außendurchmesser der Zwischenwärmetauscher erreicht, daß die Zwischenwärmetauscher und die Katalysatorschichten nicht mehr unbedingt eine Einheit sind, sondern separat in den Konverter ein- und ausgebaut werden können. Bei einem Schaden an den Zwischenwärmetauschern oder am Katalysator kann jeder für sich aus dem Reaktorbehälter entfernt werden. Es sind keine umständlichen Katalysatorfüll- und leereinbauten durch die Wärmetauscher hindurch erforderlich.

Durch den erfindungsgemäßen Einbau der Wärmetauscher zentral in die Katalysatorschicht wird erreicht, daß die Wärmetauscher mit den jeweils optimalen Abmessungen, d.h. besten, Wärmeübertragungswerten und wirtschaftlichsten Rohrabmessungen ausgelegt werden können. Der Durchmesser der Wärmetauscher ist nicht mehr zwangsläufig an den Durchmesser des Konverters gebunden. Bekannterweise sind bei einem Wärmetauscher gewisse Beziehungen zwischen Gasgeschwindigkeiten in den Rohren und den Spaltbreiten zwischen den Rohren für optimalen Wärmeaustausch einzuhalten.

Die erfindungsgemäße Vorrichtung erlaubt weiterhin den Einbau optimaler Wärmetauscher zentral in die Katalysatorbehälter in Konverter mit großem Durchmesser von 2 m und mehr unter Vermeidung der obengenannten Schwierigkeiten. Der Verschluß kann auf einen Durchmesser eingezogen werden, der es erlaubt, einen bekannten leicht zu handhabenden Deckel zu verwenden. Dadurch ist es möglich, durch Verkürzen der Montagezeiten zu optimaler Betriebsdauer zu erlangen.

Ein Ausführungsbeispiel der Erfindung ist in der Zeichnung dargestellt und wird im folgenden beschrieben: Die Vorrichtung zur Durchführung exothermer, katalytischer Gasreaktionen für die Ammoniak- oder Methanolsynthese besteht im wesentlichen aus dem Hochdruckmantel 1 mit dem eingezogenen Deckel 2, dem Einsatz 3, der die Katalysatorbehälter 4, 4' und die Gas/Gas-Wärmetauscher 5, 5' enthält. Über die Beschreibung des Gasweges beim Betrieb der Vorrichtung erfolgt die Beschreibung der konstruktiven Verknüpfung der einzelnen Bauelemente untereinander.

Über den Gaseintrittsstutzen 6 im Hochdruckmantel 1 tritt das Umlaufgas, hier als Frischgas bezeichnet, mit niedriger Temperatur in den Hochdruckmantel ein und strömt im Ringraum zwischen Hochdruckmantel 1 und Einsatz 3 nach oben. Auf seinem Weg in diesem Ringraum kühlt es gleichzeitig den Hochdruckmantel von innen. Aus dem Freiraum 7 oberhalb des Einsatzes strömt das Frischgas in das zentrale Leitrohr 8, um wieder nach unten vor den Rohrboden 9 des ersten Gas/Gas-Wärmetauschers 5 geführt zu werden. Die Abdeckhaube 10, am unteren Rohrboden des ersten Gas/Gas-Wärmetauschers 5 leitet das aufzuheizende Frischgas in die Rohre des Gas/Gas-Wärmetauschers. Dieser erste Gas/Gas-Wärmetauscher 5 erstreckt sich zentral über die Länge des Behälters 4' für die zweite Katalysatorschicht. Der obere Rohrboden 11 bildet zusammen mit dem unteren Rohrboden 12 des zweiten Gas/Gas-Wärmetauschers 5' in dem Behälter 4 für die erste Katalysatorschicht und mit dem Mantelblech 13 eine Kammer 14 für die Temperaturregelung des bereits teilweise aufgeheizten Frischgases. In diese Kammer 14 mündet die Leitung 15, über die kaltes Frischgas dem bereits teilweise aufgeheizten Frischgas zugemischt werden kann. Aus der Kammer 14 strömt das teilweise aufgeheizte Frischgas durch die Rohre des zweiten Gas/Gas-Wärmetauschers 5' bis in den Freiraum 16 oberhalb des Behälters 4 für die erste Katalysatorschicht. In diesen Freiraum mündet eine nicht dargestellte Leitung für Kaltgas, um im Bedarfsfall durch Zugabe von Kaltgas die Temperatur des aus dem zweiten Gas/Gas-Wärmetauscher austretenden aufgeheizten Frischgases bewußt erniedrigen zu können. Das auf eine bestimmte Temperatur eingestellte Frischgas aus dem Freiraum 16 erfährt wiederum eine Richtungsänderung und strömt nach unten in den Ringraum 17 zwischen Einsatz 3 und perforierter Außenwand des Katalysatorbehälters 4. Von hieraus verteilt es sich in die Katalysatorfüllung 18, strömt radial zum Ringraum 19, um von dort nach oben in den Mantelraum des zweiten Gas/Gas-Wärmetauschers 5' zu gelangen. Das durch die exotherme Reaktion in der ersten Katalysatorfüllung stark aufgeheizte, zu Reaktionsgas gewordene Umlaufgas tritt in indirekten Wärmetausch mit dem aufzuheizenden Frischgas in den Rohren des Gas/Gas-Wärmetauschers 5'. Nach Durchströmen des Wärmetauschers gelangt der Gasstrom in den Freiraum 20, um von hier aus in den Ringraum 21 zwischen Einsatz 3 und perforierter Außenwand des Katalysatorbehälters 4' zu strömen. Die Durchströmung der Katalysatorfüllung und des Mantelraumes des ersten Gas/Gas-Wärmetauschers erfolgt nun analog zur Beschreibung für die erste Katalysatorschicht und den zweiten Gas/Gas-Wärmetauscher. Das aus dem Mantelraum des ersten Gas/Gas-Wärmetauschers 5 ausströmende, teilweise abgekühlte Reaktionsgas wird mittels der Glocke 22 gesammelt und über den Stutzen 26 aus dem Hochdruckmantel abgeführt.

Die beiden Gas/Gas-Wärmetauscher 5, 5' sind fest mit dem Leitrohr 8 verbunden, d.h. verschweißt und liegen auf dem oberen Rand der inneren Wand des unteren Katalysatorbehälters 4' auf. Von hier aus können sich beide Wärmetauscher einseitig frei ausdehnen, ohne daß es zu schädlichen Wärmedehnungen kommt. Die Abdichtung des oberen Rohrbodens 23 des zweiten Wärmetauschers und des Leitrohres am Deckel 24 des ersten Katalysatorbehälters bzw. am Einsatzdeckel erfolgt durch Stopfbuchsen in bekannter Weise.

Erfindungswesentlich ist, daß beide Wärmetauscher und das Leitrohr eine Einheit bilden und der Fixpunkt dieser Einheit in etwa in der Mitte der Einheit liegt, so daß sich beide Wärmetauscher mit ihrem heißgehenden Teil frei ausdehnen können.

## Patentansprüche

1. Vorrichtung zur Durchführung exothermer, katalytischer Gasreaktionen für die Ammoniak- oder Methanol-Synthese, im wesentlichen bestehend aus einem Hochdruckmantel (1) und einem Einsatz (3) mit oberem Abschlußdeckel (2), wobei der Einsatz mindestens zwei übereinander angeordnete Katalysatorbehälter (4,4') in ringförmiger zylindrischer Anordnung mit gasdurchlässiger Innen- und Außenwand für radiale Gasströmung von außen nach innen und zwei Gas/Gas-Wärmetauscher (5, 5) in Rohrbündelbauart zentral innerhalb des ersten und des zweiten Katalysatorbehälters enthält, gekennzeichnet durch

a) ein zentrales Leitrohr (8), das sich vom freien Raum (7) oberhalb des Einsatzes (3), gegen diesen mittels Stopfbuchse abgedichtet, bis zum unteren Rohrboden (9) des ersten, im zweiten Katalysatorbehälter zentral angeordneten Gas/Gas-Wärmetauschers (5) erstreckt,

b) eine Abdeckhaube (10) unterhalb des unteren Rohrbodens (9) zur Gasumlenkung aus dem zentralen Leitrohr (8),

c) ein Röhrenbündel des ersten Gas/Gas-Wärmetauschers (5) mit einem Wärmetauschermantel in einer Länge kürzer als das Röhrenbündel, so daß beidseitig Gasein- und Gasaustritt verbleibt und wobei der Endrohrboden (11) des ersten Gas/Gas-Wärmetauschers gasdicht und kraftschlüssig das Leitrohr (8) umschließt und gasdicht auf der Innenwand des Behälters (4') für die zweite Katalysatorschicht aufliegt,

d) eine Kammer (14), gebildet aus dem Endrohrboden (11) des ersten Gas/Gas-Wärmetauschers (5) und dem Anfangsrohrboden (12) des zweiten Gas/Gas-Wärmetauschers (5') und dem Mantelblech (13) und mit einem Zufuhrleitungsauslass (15) für Zumischgas in diese Kammer,

e) ein Röhrenbündel des zweiten Gas/Gas-Wärmetauschers (5') mit einer Länge größer als die Länge des ersten Katalysatorbehälters (4) und einem Wärmetauschermantel mit einer Länge kleiner als das Röhrenbündel, so daß beidseitig Gasein- und Gasaustritt verbleiben und einem Endrohrboden (23), wobei die Anfangsrohrbodenplatte (12) des zweiten Gas/Gas-Wärmetauschers gasdicht und kraftschlüssig das Leitrohr (8) umschließt, und wobei der Enrohrboden (23) über zwei Stopfbuchspackungen gegen das Leitrohr (8) und den Deckel (24) des ersten Katalysatorbehälters 4 verschiebbar abgedichtet ist.

2. Vorrichtung nach Anspruch 1 gekennzeichnet durch eine Zufuhrleitung (25) für Zumischgas, die das zentrale Leitrohr (8) innerhalb der ersten Katalysatorschicht ringförmig umgibt, wobei die Anfangsrohrbodenplatte (12) des zweiten Gas/Gas-Wärmetauschers die Zufuhrleitung (25) für Zumischgas gasdicht und kraftschlüssig umschließt.

## Claims

1. Device for performing exothermal catalytic gas reactions for the ammonia or methanol synthesis, comprising essentially a high-pressure shell (1), an insert (3), and an upper cover (2), the insert being equipped at least with two superimposed annular cylindrical catalyst containments (4, 4') having a gas-permeable inner and outer wall for radial gas flow from the outside towards the inside and with two tubular gas/gas heat exchangers (5, 5') arranged centrally in the first and second catalyst containment, characterized by

a) a central guide tube (8) reaching from the free space (7) above the insert (3) against which the tube is sealed by a stuffing box packing, to the lower tubesheet (9) of the first gas/gas heat exchanger (5) arranged in the centre of the second catalyst containment,

b) a bonnet (10) below the lower tubesheet (9) serving as baffle for the gas being introduced through the central guide tube (8),

c) a tube bundle of the first gas/gas heat exchanger (5) being installed in a shell shorter than the tube bundle so as to allow the gas to enter and to leave at both ends, the upper tubesheet (11) of the first gas/gas heat exchanger having a gastight and firm connection around the guide tube (8) and a gastight support on the inner wall of the containment (4') for the second catalyst bed,

d) a chamber (14) being formed by the upper tubesheet (11) of the first gas/gas heat exchanger (5), the lower tubesheet (12) of the second gas/gas heat exchanger (5'), and the shell plate (13), and being provided with a bypass line outlet (15) for the admission of make-up gas into this chamber,

e) a tube bundle of the second gas/gas heat exchanger (5') being longer than the first catalyst containment (4) and being installed in a shell shorter than the tube bundle so as to allow the gas to enter and to leave at both ends, the lower tubesheet (12) of the second gas/gas heat exchanger having a gastight and firm connection around the guide tube (8), and the upper tubesheet (23) being movably sealed by two stuffing box packings against the guide tube (8) and the cover (24) of the first catalyst containment (4).

2. Device according to claim 1, characterized by a make-up gas bypass line (25) being designed as ring line surrounding the central guide tube (8) within the first catalyst bed, the lower tubesheet (12) of the second gas/gas heat exchanger having a gastight and firm connection around the make-up gas bypass line (25).

## Revendications

1. Appareil servant à réaliser les réactions gazeuses catalytiques, exothermiques, pour la synthèse de l'ammoniac ou du méthanol, composé principalement d'une enveloppe haute-pression (1), d'un élément intérieur (3) et d'un couvercle (2) à la partie supérieure, étant entendu que ledit élément consiste en deux récipients de catalyse superposés (4, 4') de forme annulaire, cylindrique, aux parois intérieure et extérieure perforées de manière à assurer un courant gazeux radial de l'extérieur vers l'intérieur, et en deux échangeurs de chaleur gaz/gaz (5, 5') à faisceau tubulaire placés respectivement au centre du premier et du deuxième récipient de catalyse, caractérisé en ce que

a) un tube directeur central (8) descend de l'espace libre au-dessus de l'élément intérieur (3), l'étanchéité par rapport à ce dernier étant assurée au moyen d'un presse-étoupe, jusqu'à la plaque tubulaire inférieure du premier échangeur de chaleur gaz/gaz disposé au centre du récipient de catalyse;

b) une calotte (10) placée sous la plaque tubulaire inférieure (9) sert à dévier le gaz sortant du tube directeur central (8);

c) un faisceau tubulaire du premier échangeur de chaleur gaz/gaz (5) est doté d'une calandre dont la longueur est inférieure à celle du faisceau tubulaire, de sorte que le gaz entre et sort des deux côtés, et étant entendu que la plaque tubulaire d'extrémité (11) du premier échangeur de chaleur gaz/gaz enserre le tube directeur (8) de manière étanche aux gaz, par adhérence, et repose sur la paroi intérieure du deuxième récipient de catalyse (4') de manière étanche aux gaz;

d) une chambre (14) est formée par la plaque tubulaire d'extrémité (11) du premier échangeur de chaleur gaz/gaz (5), la plaque tubulaire initiale (12) du second échangeur de chaleur gaz/gaz (5') et le caisson en tôle (13) et dotée d'une sortie de conduite d'amenée pour le gaz d'apport (15);

e) un faisceau tubulaire du deuxième échangeur de chaleur gaz/gaz (5'), dont la longueur est supérieure à celle du premier récipient de catalyse (4) est doté d'une calandre plus courte que le faisceau tubulaire, de sorte que le gaz entre et sort des deux côtés, ainsi que d'une plaque tubulaire d'extrémité (23), étant entendu que la plaque tubulaire initiale (121) du second échangeur de chaleur gaz/gaz enserre le tube directeur (8) de manière étanche aux gaz, par adhérence, et que la plaque tubulaire d'extrémité (23) est étanchée par rapport au tube directeur et au couvercle du premier récipient de catalyse (4) au moyen de deux garnitures à presse-étoupe.

2. Appareil selon la revendication 1 caractérisé en ce qu'une conduite d'amenée (25) de gaz d'apport, encercle le tube directeur central (8) au centre du premier lit de catalyse, étant entendu que la plaque tubulaire initiale du deuxième échangeur de chaleur gaz/gaz enserre la conduite d'amenée de gaz d'apport de manière étanche aux gaz, par adhérence.